# EUROPEAN PATENT APPLICATION

(11) **EP 4 471 796 A1**
(43) Date of publication of application: **04.12.2024**
(21) Application number: 23176071.1
(22) Date of filing: 30.05.2023
(51) Int. Cl.: G16H 50/20, G06N 20/00, G16H 50/70

(54) **IDENTIFYING A CAUSE OF INACCURACY OF A MACHINE-LEARNING ALGORITHM**

(71) Applicant: Koninklijke Philips N.V., 5656 AG Eindhoven (NL)
(72) Inventor: KELLY, Declan Patrick, 5656 AG Eindhoven (NL); HELAOUI, Rim, 5656 AG Eindhoven (NL)
(74) Representative: Philips Intellectual Property & Standards

(57) **Abstract**

A mechanism for identifying contraindications for processing of subject data in a (data) record using a machine-learning method. Medical data in historic records are processed by the machine-learning method to identify predicted output data. The predicted output data is compared to corresponding data in the historic records to flag or otherwise identify historic records where there is a mismatch. The flagged historic records are then processed to identify any shared elements therebetween, which thereby represent contraindications for processing subject data using the machine-learning method.

## Description

### FIELD OF THE INVENTION

The present invention relates to the field of machine-learning, and in particular to the application of machine-learning on medical data.

### BACKGROUND OF THE INVENTION

There is a growing interest in using machine-learning methods to process some input medical data (of a clinical subject) to produce output medical data. The processing can be performed, for instance, to predict one or more values for parameters of the clinical subject, to predict the presentation of pathologies, to perform segmentation on medical images, to generate recommendations for treatment of the clinical subject and so on.

However, although generally considered relatively reliable, machine-learning methods are not foolproof. There is therefore a desire to facilitate the identification of when a machine-learning method is inaccurate or is likely to be inaccurate.

### SUMMARY OF THE INVENTION

The invention is defined by the claims.

According to examples in accordance with an aspect of the invention, there is provided a computer-implemented method for identifying one or more potential causes of inaccuracy for a machine-learning method configured to process input medical data to produce predicted output medical data.

The computer-implemented method comprises: receiving a plurality of historic records, each associated with a different clinical subject and/or a subject at different points in time, each historic record containing subject data including: historic input medical data for input to the machine-learning method; and historic output medical data representing a ground-truth version of the predicted output medical data for the historic input medical data. Thus, the historic output medical data represents a ground-truth version of the predicted output medical data that would be generated if the historic input medical data were processed using the machine-learning method.

The computer-implemented method comprises, for each historic record: processing a portion of the historic input medical data using the machine-learning algorithm to produce predicted output medical data for the historic record; and flagging the historic record only when it is determined that a difference between the predicted output medical data and the historic output medical data meets one or more predetermined criteria.

The computer-implemented method comprises processing all flagged historic records to identify one or more elements of subject data common to more than a predetermined percentage or number of the subjects associated with the flagged historic records.

The identified one or more elements thereby act as contraindications for using the machine-learning method, e.g., one or more values for particular parameters contained in medical data that indicate that the machine-learning method should not be used to process the medical data of that subject.

It has been herein recognized that the nature of a machine-learning method is such that it is not possible or practical to train a machine-learning method to work in all scenarios due to limited data. Thus, there may be a subset of a population whose medical data, if processed using the machine-learning method, will not produce accurate output medical data.

It has also been recognized that there such a subset may share certain characteristics (e.g., certain ages, certain medical histories, certain medications and so on). Any shared characteristics can therefore act as a contraindication for using the machine-learning method.

This information can be advantageously used to avoid use of the machine-learning method if a future subject has the same contraindications, to avoid generation of inaccurate information used for making a clinical decision. Alternatively, this information could be used to train or retrain the machine-learning method to be suited for processing medical data of such subject, to finetune the machine-learning method.

The machine-learning method may, for instance, be any machine-learning method that produces output data upon which a clinical decision can be made. Thus, the machine-learning method is configured to output relevant data for a clinician to make a clinical decision.

A ground-truth version of the predicted output medical data is the actually recorded version of the predicted output medical data that is produced by the machine-learning method. Of course, if the machine-learning method generates a probability of an event or the like, then the ground-truth version may be an indicator of whether said event actually occurred (according to the historic record). Such data is considered to be a ground-truth version of the predicted probability by the machine-learning algorithm.

In some examples, the subject data includes values for a plurality of different properties of the clinical subject; and the one or more elements comprise at least one value or value range for the one or more different properties. This approach advantageously facilitates ease of identifying common shared elements between different clinical subjects associated with flagged historic records.

The one or more elements may be able to comprise at least one element of the subject data not present in the historic input medical data. In particular, the one or more elements may comprise at least one element of the subject data not present in the historic input medical data. This approach advantageously looks for contraindications that are not present in the data processed by the machine-learning method, such that the machine-learning method may not have been trained to take account of such differences. This advantageously facilitates identification of a weakness or inaccuracy of the machine-learning method.

In some examples, the step of processing the flagged historic records comprises processing all historic records to identify one or more elements of subject data common to more than the predetermined percentage or number of the subjects associated with the flagged historic records and common to less than a second predetermined percentage of the subjects associated with the historic records.

This approach avoids identifying elements that are particularly representative of the flagged historic records, rather than being characteristics of the overall population (represented by the historic records). This improves the identification of potential contraindications for use of the machine-learning method.

In some examples, the number of subjects, in the predetermined percentage or number of the subject associated with the flagged historic records, is less than the number of subject associated with the second predetermined percentage of the subject associated with the historic records.

In some examples, the predicted output medical data is a predicted value for a parameter of the subject. Thus, the machine-learning method may be configured to process input medical data to predict a value for a parameter of the subject. This facilitates quantifying comparisons, as well as improved ease of comparison, between a predicted value for the parameter and the recorded (historic) value for the parameter.

The subject data may comprise demographic information of the clinical subject. In some examples, the identified one or more elements comprises one or more demographic elements containing demographic information of the clinical subject. It is recognized that demographic information is more likely to comprise (hidden) contraindications for a machine-learning method than other forms of subject data. In particular, the range of demographics that are used in training a machine-learning method are typically limited or restricted, e.g., as they may be trained using data from a particular country or jurisdiction (e.g., which may be different to a country of deployment of the machine-learning method). By including demographic information, contraindications for local demographics can be identified as used to advantage.

The plurality of historic records may be historic records for a single clinical location. This approach allows for identification of local or specific contraindications that may not be present in a global enviroment. This improves the relevance of the identified shared or common elemetns for a particualr environment.

The predetermined percentage or number may be a predetermined percentage of no less than 10%. However, other percentages will be readily apparent to the skilled person and may depend upon local conditions, use case scenarios and/or a desired accuracy or false error rate. For instance, the predetermined percentage may be no less than 5%, no less than 10%, no less than 15% or no less than 20%. In some examples, the predetermined percentage is no more than 50%, e.g., no more than 40%, e.g., no more than 30%.

The predetermined percentage or number may be a predetermined number of no less than 50. However, other numbers will be readily apparent to the skilled person and may depend upon local conditions, number of historic records, use case scenarios and/or a desired accuracy or false error rate. For instance, the predetermined number may be no less than 20, e.g., no less than 50, e.g., no less than 100, e.g., no less than 200. The predetermined number may be no greater than 1000, e.g., no greater than 500.

There is also proposed a computer-implemented method of training a machine-learning method configured to process input medical data to produce predicted output medical data. The computer-implemented method comprises performing the computer-implemented method previously described; and retraining the machine-learning method using at least some of flagged historic records.

This provides customization and improved training of the machine-learning algorithm, such that it can be trained to be more accurate in processing input medical data of subjects that previously may have been inaccurately processed, i.e., those subjects having contraindications as previously identified.

Preferably, at least some of the flagged historic records includes at least the flagged historic records that contain any of the one or more common elements of subject data. This improves the accuracy of the machine-learning algorithm for specific contraindications, i.e., improves the performance of the machine-learning algorithm for those subjects falling within potential contraindications.

There is also provided a computer-implemented method of controlling the processing of input subject data. The computer-implemented method comprises: receiving each identified element of subject data resulting from the performance of a previously described method for identifying one or more potential causes of inaccuracy for a machine-learning method.

The computer-implemented method also comprises processing the input subject data to determine whether or not the input subject data contains any of the identified elements of subject data; and preventing the input subject data from being processed using the machine-learning method responsive to determining that the input subject data contains any of the identified elements of subject data.

The computer-implemented method may further comprise permitting the input subject data to be processed using the machine-learning method responsive to determining that the input subject data does not contains any of the identified elements of subject data.

There is also proposed a computer program product comprising computer program code means which, when executed on a computing device having a processing system, cause the processing system to perform all of the steps of any herein disclosed method.

There is also provided a processing system for identifying one or more potential causes of inaccuracy for a machine-learning method configured to process input medical data to produce predicted output medical data.

The processing system is configured to receive a plurality of historic records, each associated with a different clinical subject and/or a subject at different points in time, each historic record containing subject data including: historic input medical data for input to the machine-learning method; and historic output medical data representing a ground-truth version of the predicted output medical data for the historic input medical data;; for each historic record: process the historic input medical data using the machine-learning algorithm to produce predicted output medical data for the historic record; and flag the historic record only when it is determined that a difference between the predicted output medical data and the historic output medical data meets one or more predetermined criteria; and process all flagged historic records to identify one or more elements of subject data common to more than a predetermined percentage or number of the subjects associated with the flagged historic records.

For each historic record, the historic output medical data represents a ground-truth version of the predicted output medical data that would be generated if the historic input medical data were processed using the machine-learning method.

The skilled person would be readily capable of producing or adapting a processing system for performing any herein described method, and vice versa.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flowchart illustrating a proposed method;
Fig. 2 is a flowchart illustrating another proposed method; and
Fig. 3 is a flowchart illustrating yet another proposed method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for identifying contraindications for processing of subject data in a (data) record using a machine-learning method. Medical data in historic records are processed by the machine-learning method to identify predicted output data. The predicted output data is compared to corresponding data in the historic records to flag or otherwise identify historic records where there is a mismatch. The flagged historic records are then processed to identify any shared elements therebetween, which thereby represent contraindications for processing subject data using the machine-learning method.

Embodiments are based on the realization that historic records will provide real life examples of data for which the accuracy of a machine-learning method can be assessed. In particular, the historic records can be processed to identify contraindications for a machine-learning method.

Proposed approaches can be employed in any clinical environment in which a plurality of historic data records about different clinical subject is/are stored. Examples include, but are not limited to, hospitals, clinics, walk-in centers, emergency rooms doctor's surgeries and so on.

In the context of the present disclosure, a historic record is a historic data record in that it is a record or data structure that contains data. An example of a suitable historic record is an electronic medical record.

The present disclosure relates to the field of machine-learning methods, and in particular to machine-learning methods that process input medical data in order to produce output medical data.

A machine-learning algorithm is any self-training algorithm that processes input data in order to produce or predict output data. Here, the input data comprises input medical data and the output data comprises output medical data. The output medical data is any data suitable for use by a clinician in making a clinical decision, and can vary depending upon the environment or use-case scenario.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example instances of input medical data. The training output data entries correspond to example instances of output medical data.

The present invention recognizes that the training dataset used to train a machine-learning method (at least in the medical space) typically contain a limited selection of relevant parameters that are used to train a model to predict an outcome. Normally the training dataset does not contain the entirety of medical subject's health records, as least for the sake of privacy. This can introduce error into the machine-learning method, as not all information can be taken into account.

Moreover, the training dataset will typically be of a limited size (e.g., have a limited number of training input/output data entries). This may be due to access restrictions, privacy restrictions, licensing restrictions and so on. Thus, a typical machine-learning algorithm is unlikely to be trained to cover all possible scenarios or types of medical subject. By way of example only, this is particularly true when it comes to sensitivity in machine-learning methods configured to predict anomalies and deteriorations. Such machine-learning methods suffer significantly from the scarcity of training dataset that covers clinically relevant events, as those are usually highly under-represented compared to normal events. Examples include cardiac abnormalities from ECG data, monitoring alarms in general, deterioration prediction, warning scores etc.

Such limited datasets, e.g., limited by the number of relevant parameters or number of entries in the training dataset, carry the risk that a machine-learning method will be trusted, even if it is not accurate across an entire population.

Moreover, even appropriately trained machine-learning methods do not, in practice, achieve perfect prediction. For example, a machine-learning method with 99% accuracy is considered to have excellent performance, but will still be expected to make an incorrect prediction in 1 out of every 100 medical subjects on average.

The present disclosure provides a technique for identifying a potential cause of inaccuracy in a machine-learning method (trained to process medical data). More particular, the proposed technique facilitates identification of elements of medical data that indicate that the machine-learning method is unlikely to be accurate. Thus, the proposed technique identifies one or more contraindications for using the machine-learning method to process the medical data.

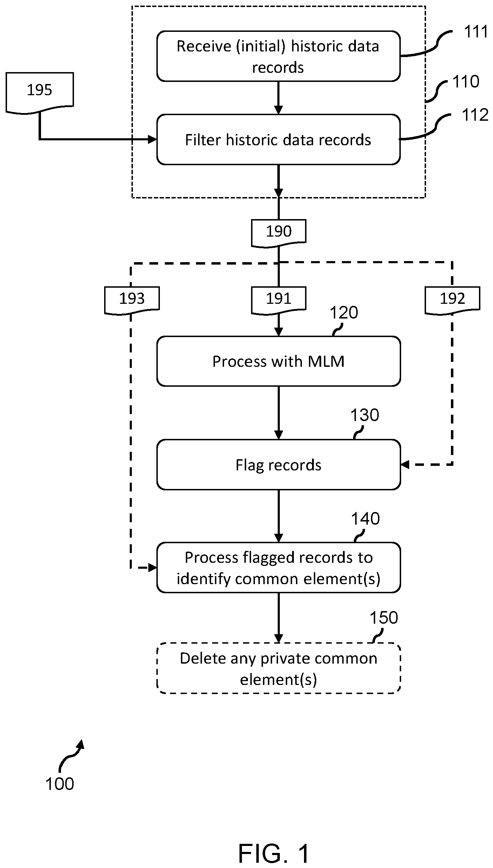

Fig. 1 is a flowchart illustrating a method 100 according to an embodiment.

The method 100 is for identifying one or more potential causes of inaccuracy for a machine-learning method. In particular, method 100 is configured to identify data elements that are shared or common between historic records of different subjects that, when processed, produce inaccurate results. This effectively aims to identify any contraindications for use of the machine-learning method.

As previously mentioned, the machine-learning method is configured to process input medical data to produce predicted output medical data.

The method 100 comprises a step 110 of receiving a plurality of historic records, each associated with a different clinical subject and/or a subject at different points in time. A historic record may, for instance, comprise an electronic medical record of a clinical subject or any other form of data record or dataset that contains data about the clinical subject.

Step 110 may be performed, for instance, by receiving, fetching or retrieving the plurality of historic records from a database or storage unit. Suitable approaches for receiving such data will be readily apparent to the skilled person.

In particular, each historic record contains subject data 190 that includes historic input medical data 191 for input to the machine-learning method. Thus, the historic input medical data is the data of the subject data that would be processed by machine-learning method. The subject data 190 also includes historic output medical data 192 that represents a ground-truth version of the predicted output medical data for the historic input medical data. Thus, the historic output medical data represents a ground-truth version of the predicted output medical data that would be generated if the historic input medical data were processed using the machine-learning method.

Of course, the subject data 190 of each historic record may contain additional subject data 193, e.g., data that is neither processed by the machine-learning method nor represents an output of a machine-learning method. By way of example, the subject data 190 may comprise demographic information (e.g., age, gender, ethnicity, nationality, location etc.), which is particularly advantageous in later examples. As a further example, the subject data 190 may comprise identifying information.

The method 100 also comprises a step 120 of, for each historic record, processing the historic input medical data using the machine-learning algorithm to produce predicted output medical data for the historic record. It will be clear that the machine-learning algorithm does not need to process all of the subject data, but rather only the historic input medical data portion of the subject data.

The method 100 also comprises a step 130 of, for each historic record, flagging the historic record only when it is determined that a difference between the predicted output medical data and the historic output medical data meets one or more predetermined criteria. In this way, step 130 flags any historic record that is identified as being inaccurately processed by the machine-learning method, such that the corresponding predicted historic output data is inaccurate.

Flagging the record may be as simple as, for instance, storing flagged historic record in a separate memory space, recording a location of the flagged historic record in a dataset or modifying a flag value carried by the historic record. Other suitable examples for marking or flagging a historic record will be readily apparent to the skilled person.

The one or more predetermined criteria may, for instance, depend upon the specific use case scenario and/or the type of output data produced by machine-learning method.

For instance, if the machine-learning method predicts a value for a physiological parameter, then the one or more predetermined criteria may be that a difference between the value provided by the predicted historic output data and the value of ground-truth version is more than a predetermined percentage, e.g., 10%, of the greater of the two values.

As another example, if the machine-learning method performs segmentation of a particular anatomical element, then the one or more predetermined criteria may be that a total overlap between the two segmentation results (predicted and ground-truth) is less than a predetermined percentage of the one of the two segmentation results.

As yet another example, if the machine-learning method produces a discharge readiness score (e.g., estimating a risk of death or readmission within 48 hours of discharge) and the ground-truth version indicates an actual result of death or readmission within 48 hours of discharge (e.g., which would indicate a value at either end of the discharge readiness score scale), then the one or more predetermined criteria may include a criteria that the difference between the discharge readiness score and the ground truth version exceeds a predetermined value (e.g., half the total range of the discharge readiness score).

As a further example, if a machine-learning method is configured to predict a diagnosis or condition, e.g., based on a medical image such as a CT scan or X-ray, then the one or more predetermined criteria may be that the predicted diagnosis is semantically equivalent to the ground-truth diagnosis.

A wide variety of other suitable examples will be readily apparent to the skilled person.

More generally, each predetermined criterion may be a criterion that indicates a reliability or accuracy of the predicted historic output data. More particularly, the one or more predetermined criteria may include any criteria that indicates that the predicted historic output data is inaccurate for the purposes of making an accurate and reliable clinical decision.

The method 100 also comprises a step 140 of processing all flagged historic records to identify one or more elements of subject data common to more than a predetermined percentage or number of the subjects associated with the flagged historic records. The subject data is not restricted to only the input and/or output historic medical data, but may include all subject data found in each flagged historic record. Thus, the one or more elements is able to comprise at least one element of the subject data not present in the historic input medical data.

Put another way, step 140 effectively comprises using all the subject data found in flagged historic record (e.g., including data not used by the machine-learning method) to look for commonalities between failure cases as potential contraindications.

In this way, step 140 aims to identify common or shared parts of subject data between different flagged historic records. In this way, step 140 identifies potential contraindications for the machine-learning method, as it identifies elements of subject data that are common amongst clinical subjects for which the machine-learning method performs inaccurate prediction.

A shared or common element may be any portion or part of a subject data that is the same or similar for different historic records that have been flagged (as being inaccurately processed by the machine-learning method). In particular, the shared/common element may be a label for a value or values of subject data that are shared by historic records that are inaccurately processed.

Embodiments are particularly advantageous when the one or more shared element(s) comprises, or is permitted to comprise, shared elements between demographic data present in subject data of each historic medical record. This allows for identification of contraindications that may not be otherwise available.

Thus, the identified one or more elements may comprise one or more demographic elements containing demographic information of the clinical subject.

The predetermined percentage or number may vary depending upon the use-case scenario. However, as a working example, the predetermined percentage or number may be a predetermined percentage of no less than 10%. As an alternative working example, the predetermined percentage or number is a predetermined number of no less than 50. The skilled person will readily appreciate how this/these value(s) may change or differ depending upon the use or need.

For instance, if used, the predetermined percentage may be no less than 5%, no less than 10%, no less than 15% or no less than 20%. In some examples, the predetermined percentage is no more than 50%, e.g., no more than 40%, e.g., no more than 30%.

If used, the predetermined number may depend upon local conditions, number of historic records, use case scenarios and/or a desired accuracy or false error rate. For instance, the predetermined number may be no less than 20, e.g., no less than 50, e.g., no less than 100, e.g., no less than 200. The predetermined number may be no greater than 1000, e.g., no greater than 500.

By way of example, in some embodiments the subject data includes values for a plurality of different properties of the clinical subject. The one or more elements (identified in step 140) may comprise at least one value or value range for the one or more different properties. This approach facilitates identification of elements (i.e., values or range of values) that are shared between different subjects having flagged historic data records.

One approach to performing step 140 can be to apply a clustering algorithm to the flagged historic records. A clustering algorithm will cluster similar flagged historic records together. Each cluster may then be assessed to identify the cause(s) of the similarity of records within the respective cluster.

Step 140 thereby identifies one or more elements of subject matter that are common to more than a predetermined percentage or number of the subjects associated with the flagged historic records. Thus, the identified one or more elements of subject matter are predicted contraindications to using the (current version of the) machine-learning method.

One technique to improve the performance of step 140 is determine whether or not the identified one or more elements of subject matter are not common to a second predetermined percentage of the historic records. In particular, if an identified element of subject matter is common to more than a second predetermined percentage of the historic records, then it may be ignored, discarded or discounted as an identified element of subject matter.

This approach recognizes that a desirable function of step 140 is to identify commonalities that occur in higher proportion in the flagged historic records vs the total population, indicating that the error rate is higher for the flagged historic records.

Accordingly, step 140 may comprise processing all historic records (including the flagged historic records) to identify one or more elements of subject data common to more than a predetermined percentage or number of the subjects associated with the flagged historic records and common to less than a second predetermined percentage of the subjects associated with the historic records.

The number of subjects, in the predetermined percentage or number of the subject associated with the flagged historic records, may be less than the number of subject associated with the second predetermined percentage of the subject associated with the historic records.

Thus, if used, the second predetermined percentage is less than: the predetermined percentage P_{P}; a percentage of the flagged historic records represented by the predetermined number (i.e., a percentage P_{PN}, where P_{PN} = 100.N_{P}/N_{FR}, where N_{P} is the predetermined number and N_{FR} is the number of flagged records); a percentage of the flagged historic records for which the element of subject data is common (i.e., a percentage P_{FR}, where P_{FR} = 100.N_{CR}/N_{FR}, where N_{CR} is the number of flagged historic records having the common element of subject data and N_{FR} is the total number of flagged records).

The second predetermined percentage may be less than half of: the predetermined percentage; the percentage of the flagged historic records represented by the predetermined number; or the percentage of the flagged historic records for which the element of subject data is common.

The second predetermined percentage may be less than a quarter of: the predetermined percentage; the percentage of the flagged historic records represented by the predetermined number; or the percentage of the flagged historic records for which the element of subject data is common.

The identified common element(s) in step 140 can be used to multiple advantages. One example usage is to caution or prevent the processing of future data records containing any of the common element. Another example usage is to guide or control future training of the machine-learning method such that it more accurately processes data records containing any such common element.

In particular, future training of the machine-learning method can be performed to improve the performance of a single machine-learning method that is applied to all populations, or to improve the performance of a machine-learning method specifically for those subjects having the contraindications to the original machine-learning method. Thus, a spin-off machine-learning method could be trained. If this approach is taken, in future processing of a historic record, the historic record can be processed to decide which (if any) of the machine-learning models should be applied to that user.

Other example uses will be apparent to the skilled person.

Fig. 1 illustrates some further optional steps or variants for the method 100.

As one example, the step 110 of receiving a plurality of historic records may comprise a step 111 of receiving a plurality of initial historic records and a step 112 of filtering the plurality of initial historic records to produce the plurality of historic records.

The filtering in step 112 may be performed, for instance, to target certain demographics or types of clinical subject (e.g., cardiac subjects or pediatric subjects). This may be particularly advantageous for facilitating guided identification or confirmation of potential contraindications, e.g., if a clinician suspects that a machine-learning method is inaccurate for particular subsets of a patient population.

As another example, the filtering in step 112 may be performed to filter for historic records representing a certain time period.

The filtering in step 112 may be responsive to an indication 195 of a filter to be applied. The indication 195 may, for instance, be provided by a user in the form of a user input, e.g., receive at a user interface.

The method 100 may comprise a step 150 of deleting any common element that falls within one or more predetermined categories. Deletion of some common elements may be necessary to avoid privacy risks. For instance, common elements between identifying information of individuals (e.g., specific birth dates or names) may be deleted. However, this step is not essential, and can be omitted.

In the method 100, the historic records may represent historic records for medical subjects housed and/or treated in a same clinical location. Thus, the plurality of historic records may be historic records for a single clinical location, e.g., a single hospital, clinic, surgery and so on.

Such an approach can be used to generate or produce a location specific set of possible common elements and/or contraindications. This could, for instance, be useful for identifying location-specific contraindications that may be masked or hidden over an extremely large and diverse population. This technique appreciates that a location of a subject will have an impact on the success of machine-learning method.

In another embodiment, for method 100, the historic records may represent medical subjects from many (e.g. hundreds) clinical locations or environments, which are combined such the expected real-world model performance of the machine-learning method (across different patient populations) can be determined and contraindications identified.

It is recognized that the processing of a large number of historic records may require significant processing power. Advantageously, the proposed method may only be performed or implemented during low demand times of a processing system, e.g., during night shifts if the processing system is a processing system for a clinical environment such as a hospital or clinic. This is because it is recognized that the proposed method, whilst advantageous, i.e., non-urgent from a clinical point of view.

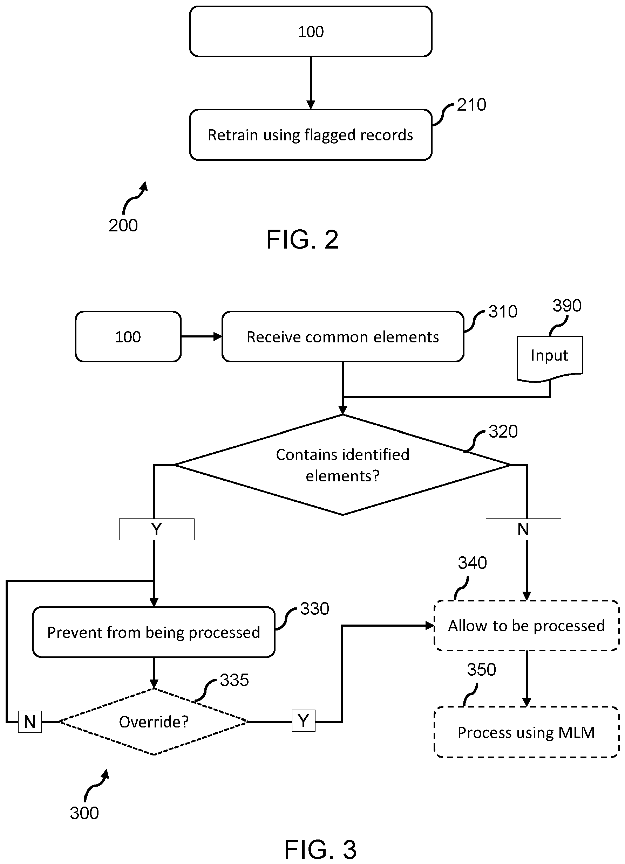

Fig. 2 illustrates a computer-implemented method 200 according to an embodiment. The computer-implemented method 200 illustrates techniques for training a machine-learning method. As previously mentioned, the machine-learning method is configured to process input medical data to produce predicted output medical data.

The method 200 comprises performing the method 100 previously described.

The method 200 then moves to a step 210 of retraining the machine-learning method using at least some of flagged historic records. In this way, the flagged historic records that represent errors in the machine-learning method are used to train the machine-learning method.

The step 210, i.e., the retraining step, may use at least the flagged historic records that contain any of the one or more common elements of subject data. In other words, flagged historic data records that relate to clinical subjects having any of the one or more common elements of subject data identified in method 100 can be used to retrain the machine-learning algorithm. This facilitates training of the machine-learning algorithm to have improved performance in processing further records having the common subject data.

In some examples, the retraining step 210 does not use any of the historic records that have not been flagged during the course of method 100. This approach avoids further, potentially unnecessary, training of the machine-learning record to perform a task that it is ready suitably accurate at carrying out.

In other approaches, the retraining step 210 uses both flagged historic records and other (unflagged) historic records. In particular, the retraining step may use any or all historic records and other records that contain the common element(s) of subject data identified in method 100. This can avoid or reduce a risk of overtraining for the particular clinical subjects having the shared common element(s).

Fig. 3 illustrates a computer-implemented method 300 of controlling the processing of input subject data. It will be appreciated that the input subject data contains at least input medical data suited for being processing by the machine-learning method, but may also contain additional subject information (e.g., demographic information).

The computer-implemented method 300 comprises receiving 310 each identified element of subject data resulting from the performance of the method 100 previously described. Thus, the method 300 may comprise, for instance, performing the method 100 or simply retrieving or receiving any identified common element(s) from a memory, data store or other processing system that performs the method 100.

The computer-implemented method 300 also comprises a step 320 of processing the input subject data to determine whether or not the input subject data contains any of the identified elements of subject data.

Thus, the input subject data is checked to see whether it relates to a medical subject having any of the identified element(s) of subject data. In this way, step 320 effectively checks for any contraindications (identified in method 100) in the subject data containing data that is to be processed using the machine-learning method.

Responsive to determining that the input medical data contains any of the identified elements of subject data., the method 300 performs a step 330 of preventing the input medical data from being processed using the machine-learning method.

In some examples, step 330 may comprise providing a user-perceptible output (e.g., at a user interface). This advantageously provides an operator or user with an indicator that the machine-learning method that processes the input medical data will be inaccurate.

Of course, it may be possible for a user to override the prevention performed by step 330, to execute the machine-learning method. Thus, the method 300 may comprise a step 335 of determining whether there is an override signal. Responsive to the override signal, the input signal data the method 300 may allow (represented by step 340) the input subject data to be processed using the machine-learning method. Accordingly, the method may comprise performing a step 350 of processing input medical data contained in the input subject data using the machine-learning method.

Responsive to determining that the input medical data does not contain any of the identified elements of subject data, the method 300 may allow (represented by step 340) the input subject data to be processed using the machine-learning method. Accordingly, the method may comprise performing a step 350 of processing input medical data contained in the input subject data using the machine-learning method.

If performed, step 350 may comprise providing a user-perceptible output of the output medical data produced by the machine-learning method.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A computer-implemented method for identifying one or more potential causes of inaccuracy for a machine-learning method configured to process input medical data to produce predicted output medical data, the computer-implemented method comprising:
receiving a plurality of historic records, each associated with a different clinical subject and/or a subject at different points in time, each historic record containing subject data including:
historic input medical data for input to the machine-learning method; and
historic output medical data representing a ground-truth version of the predicted output medical data for the historic input medical data;
for each historic record:
processing a portion of the historic input medical data using the machine-learning algorithm to produce predicted output medical data for the historic record; and
flagging the historic record only when it is determined that a difference between the predicted output medical data and the historic output medical data meets one or more predetermined criteria; and
processing all flagged historic records to identify one or more elements of subject data common to more than a predetermined percentage or number of the subjects associated with the flagged historic records.

2. The computer-implemented method of claim 1, wherein:
the subject data includes values for a plurality of different properties of the clinical subject; and
the one or more elements comprise at least one value or value range for the one or more different properties.

3. The computer-implemented method of claim 1 or 2, wherein the one or more elements is able to comprise at least one element of the subject data not present in the historic input medical data.

4. The computer-implemented method of any of claims 1 to 3, wherein the step of processing the flagged historic records comprises processing all historic records to identify one or more elements of subject data common to more than the predetermined percentage or number of the subjects associated with the flagged historic records and common to less than a second predetermined percentage of the subjects associated with the historic records.

5. The computer-implemented method of claim 4, wherein the number of subjects, in the predetermined percentage or number of the subject associated with the flagged historic records, is less than the number of subject associated with the second predetermined percentage of the subject associated with the historic records.

6. The computer-implemented method of any of claims 1 to 5, wherein the predicted output medical data is a predicted value for a parameter of the subject.

7. The computer-implemented method of any of claims 1 to 6, wherein the subject data comprises demographic information of the clinical subject.

8. The computer-implemented method of any of claims 1 to 7, wherein the identified one or more elements comprises one or more demographic elements containing demographic information of the clinical subject.

9. The computer-implemented method of any of claims 1 to 8, wherein the plurality of historic records are historic records for a single clinical location.

10. A computer-implemented method of training a machine-learning method configured to process input medical data to produce predicted output medical data, the method comprising:
performing the method of any of claims 1 to 9; and
retraining the machine-learning method using at least some of flagged historic records.

11. The computer-implemented method of claim 10, wherein the at least some of the flagged historic records includes at least the flagged historic records that contain any of the one or more common elements of subject data.

12. A computer-implemented method of controlling the processing of input subject data, the computer-implemented method comprising:
receiving each identified element of subject data resulting from the performance of the method of any of claims 1 to 9;
processing the input subject data to determine whether or not the input subject data contains any of the identified elements of subject data; and
preventing the input subject data from being processed using the machine-learning method responsive to determining that the input subject data contains any of the identified elements of subject data.

13. The computer-implemented method of claim 12, further comprising permitting the input subject data to be processed using the machine-learning method responsive to determining that the input subject data does not contains any of the identified elements of subject data.

14. A computer program product comprising computer program code means which, when executed on a computing device having a processing system, cause the processing system to perform all of the steps of the method according to any of claims 1 to 13.

15. A processing system for identifying one or more potential causes of inaccuracy for a machine-learning method configured to process input medical data to produce predicted output medical data, the processing system being configured to:
receive a plurality of historic records, each associated with a different clinical subject and/or a subject at different points in time, each historic record containing subject data including:
historic input medical data for input to the machine-learning method; and
historic output medical data representing a ground-truth version of the predicted output medical data for the historic input medical data;
for each historic record:
process the historic input medical data using the machine-learning algorithm to produce predicted output medical data for the historic record; and
flag the historic record only when it is determined that a difference between the predicted output medical data and the historic output medical data meets one or more predetermined criteria; and
process all flagged historic records to identify one or more elements of subject data common to more than a predetermined percentage or number of the subjects associated with the flagged historic records.
